# NEW EUROPEAN PATENT SPECIFICATION

(11) **EP 4 125 582 B2**
(45) Date of publication and mention of the opposition decision: **19.08.2026**
(45) Mention of the grant of the patent: 24.01.2024
(21) Application number: 21716998.6
(22) Date of filing: 30.03.2021
(51) Int. Cl.: A61B 5/15, A61B 5/151

(54) **CAPILLARY BLOOD SAMPLING**
KAPILLARBLUTENTNAHME
ÉCHANTILLONNAGE DE SANG CAPILLAIRE

(30) Priority: 31.03.2020 GB 202004718
(43) Date of publication of application: 08.02.2023
(73) Proprietor: Osler Diagnostics Limited, Oxford OX1 1JD (GB)
(72) Inventor: KEW, Michael, Oxford, OX1 1JD (GB)
(74) Representative: Cousens, Nico
(86) International application number: PCT/EP2021/058366
(87) International publication number: WO 2021/198299

(56) References cited:
- WO-A1-2018/039307
- WO-A1-2018/090027
- WO-A1-2019/010008
- WO-A1-2019/226754
- WO-A2-2009/081405
- CN-U- 209 377 586
- US-A1- 2015 351 676
- US-A1- 2018 220 944

## Description

The present disclosure relates to capillary blood sampling.

### Background

Capillary blood sampling refers to sampling blood from a puncture at a site that typically includes a finger, heel or earlobe.

A typical current approach to capillary blood sampling comprises (i) wiping the site with an alcohol wipe, (ii) using a lancet to puncture the site, (iii) wiping away a first drop of blood on a piece of cotton wool, and (iv) applying blood from the site to a transfer device such as a capillary tube. Sometimes to obtain enough blood the area around the site, for example the finger, can be massaged to "milk" or express the blood. Once in the transfer device, the blood can be analysed, for example by transferring the blood to a sample chamber of a test device. The sample chamber may be part of a cartridge for use with a diagnostic system.

The present disclosure seeks to provide improvements relating to capillary blood sampling.

WO 2018/090027 A1 in the abstract states: "A blood collection device includes a housing and an interface. The blood collection device includes at least one blood collection container engaged with the interface and at least one lancet coupled to the interface. The at least one lancet extends or is configured to extend from the interface. The at least one lancet is configured to pierce tissue. The blood collection device also includes an absorbent material integrated with the housing. The absorbent material is configured to absorb fluids emanating from the tissue after the at least one lancet pierces the tissue."

WO 2019/010008 A1 in the abstract states: "A biological fluid collection device that includes a housing and a cartridge that is removably receivable within a portion of the housing is disclosed. The biological fluid collection device of the present disclosure allows for collection of capillary blood from a finger stick and provides a closed system that reduces the exposure of a blood sample. In one embodiment, a cartridge of the present disclosure also provides fast mixing of a blood sample with a sample stabilizer. In another embodiment, a cartridge of the present disclosure provides automatic plasma separation of the blood sample. Advantageously, once the cartridge is filled with a sample and removed from the housing, the cartridge can be used for a variety of important purposes."

US 2015/351676 A1 in the abstract states: "A self-contained universal front-end automatic blood collection device is disclosed herein. The device collects a few micro-liters blood sample that is used for a broad range of blood analyzers. The design of the front-end closely mimics current phlebotomist practice and provides a number of unique features that ensure patient safety and sterility, improve collection efficacy, and prevent sample contamination and user cross contamination."

WO 2018/039307 A1 in the abstract states: "A collection device (10) which directs a flow of blood into a container (14) and provides a controlled blood flow path that ensures blood flow from a collection site to a collection container is disclosed."

### Summary

This summary introduces concepts that are described in more detail in the detailed description. It should not be used to identify essential features of the claimed subject matter, nor to limit the scope of the claimed subject matter.

The present disclosure recognises that there are barriers to the adoption of capillary blood sampling, particularly when self-performed. There are too many consumables to use (alcohol wipe, lancet, cotton wool, and transfer device), the user may have a fear of sight of blood, and there may be a fear of pain. Furthermore, the typical current approach to capillary blood sampling is a complicated, multi-step procedure in which it can be difficult to hold the finger and the lancet, then the transfer device and finger with the blood drop.

The present disclosure also recognises that there are barriers to obtaining a good quality blood sample, particularly when self-performed. These include not using an alcohol wipe to clean the site before puncturing, variations in lancet size in width or depth resulting in not enough or too much blood and potentially unnecessary pain, no wiping away of the first drop of blood meaning the sample can include cell tissue contaminants, and not enough blood being introduced into the transfer device to run all tests.

According to a first aspect of the present invention, there is provided a finger lancing device comprising: a body having a chamber for receiving a finger; and a lancing device comprising a lancing member actuator for actuating a lancing member to lance a finger received in the chamber, wherein the body is flexible and made from an elastomeric material.

Advantageously, a simplified finger lancing device is provided. Furthermore, a better quality blood sample may be obtained as the lancet size and usage can be more controlled. Also, the lancet can be obscured from view as can the puncture site which can help with fear of pain or fear of the sight of blood.

The body having a chamber may be considered as an elongate body and the chamber may be considered as an elongate chamber. The body having a chamber may be considered as an elongate receptacle or an elongate sheath. The chamber/elongate chamber/elongate receptacle/elongate sheath is for receiving a finger within it. The chamber can be considered as a chamber configured to receive a finger.

The finger lancing device further comprises the lancing member, wherein the lancing member actuator is configured to actuate the lancing member to lance the finger inserted into the chamber.

The finger lancing device may further comprise a wipe provided within the chamber for wiping the finger before lancing. The wipe may be an antibacterial wipe. The wipe may be an alcohol wipe such as a 70% alcohol wipe. Advantageously, the procedure is simplified as wiping of the finger before puncturing the site is more assured.

The wipe may be provided on an inner wall of the chamber. The wipe is provided in a suitable position for wiping the finger, for example near the end of the chamber from which the finger is received. The wipe may be positioned so that the finger is moved across the surface of the wipe as it is inserted into the chamber. Again, the procedure is simplified as wiping of the finger before puncturing the site is more assured.

The finger lancing device may further comprise an absorbent wipe provided on an inner wall of the chamber for wiping away a first drop of blood. Advantageously, the procedure is simplified as wiping away of the first drop of blood is more assured.

The finger lancing device may further comprise an openable seal, the openable seal being at an end of the chamber for receiving the finger. Advantageously, the device can be kept free from contaminants and a sterile environment can be provided. The seal also helps prevent the antibacterial or alcohol wipe from drying out.

The wipe for wiping the finger before lancing may be provided on the openable seal, optionally on the inner side of the openable seal. Advantageously, the procedure is simplified as wiping of the finger before puncturing the site is more assured.

The finger lancing device may be considered as a blood collection device.

The device may further comprise a capillary collection device for collecting a blood sample from the finger received in the chamber. Again, the procedure is simplified as transfer of the blood into the capillary collection device is simpler.

The capillary collection device may have a fill line to indicate a volume of blood required. Advantageously, this helps ensure the correct amount of blood is collected.

The chamber may hold within it an elastomeric ring for guiding the finger. Advantageously, the elastomeric ring, which may be made from a rubber-like material, can provide an interference fit, thereby accommodating different finger sizes.

According to another aspect of the present invention, there is provided a body for a finger lancing device comprising a chamber for receiving a finger, wherein the body comprises a hole for receiving a lancing device, wherein the body is flexible and made from an elastomeric material, and wherein an absorbent wipe is provided on a wall of the chamber for wiping away a first drop of blood.

The body is flexible and made from an elastomeric material (e.g. silicone). Advantageously, the device may fit around different finger sizes. Advantageously, the finger may be massaged to "milk" or express more blood from the site. Accordingly, the correct amount of blood can be obtained. The body may be transparent. This enables the user to more easily position the finger to perform the blood collection.

The body of the finger lancing device (or blood collection device) may be provided on its own.

The body comprises a hole for receiving a lancing device. The hole can be considered as a hole or aperture configured to receive a lancing device.

The body may comprise an openable seal covering the hole for receiving the lancing device. The body may further comprise a hole for receiving a capillary collection device. The hole can be considered as a hole or aperture configured to receive a capillary collection device. The body may further comprise an openable seal covering the hole for receiving the capillary collection device. Advantageously, the seals mean keep the device free from contaminants and enable a sterile environment to be provided. The seals also help prevent the antibacterial or alcohol wipe from drying out.

The body and one or more of the lancing device and capillary collection device may be provided as a kit. Accordingly, in yet another aspect, the present invention provides a kit or kit of parts comprising a body as described and at least one of a lancing device or a capillary collection device.

Any one or more of the features disclosed herein in connection with the body when assembled with the lancing device and/or capillary collection device may be provided with the body on its own (i.e. without the lancing device and/or capillary collection device). These features include the wipe provided within the chamber for wiping the finger before lancing, the openable seal at an end of the chamber for receiving the finger, and the elastomeric ring. The body may be flexible and/or transparent.

The body can be provided on its own, with or without the seals, and can be packed in its own packaging such as a sterile package.

Advantageously, embodiments of the described devices can help address the aforementioned barriers to the adoption of capillary blood sampling and barriers to obtaining a good quality blood sample.

### Brief Description of the Drawings

Embodiments will now be described, by way of example only, with reference to the accompanying drawings, in which:
Figure 1 illustrates a finger lancing device in the form of a blood collection device;
Figure 2 illustrates a hand next to the blood collection device before the little finger of the hand is inserted into the device;
Figure 3 illustrates the hand and device with the little finger of the hand inserted into or received within the chamber of the device;
Figure 4 is a cross-sectional side view of the blood collection device;
Figure 5 is a cross-sectional perspective view of the blood collection device;
Figure 6 is a cross-sectional end view of the blood collection device using the same cross section as Figure 5;
Figure 7 shows the finger in a position where a drop of blood from the lanced site on the finger has been brought into contact with the end of the capillary collection device of the blood collection device; and
Figures 8A and 8B illustrate perspective views of the blood collection device showing an openable seal.

### Detailed Description

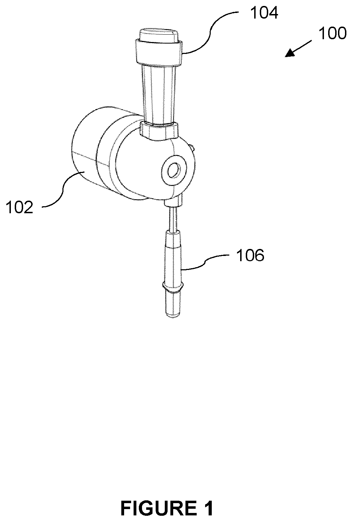

Figure 1 illustrates a finger lancing device which in this implementation is a blood collection device 100. The device 100 comprises a body 102 having a space or chamber within it for receiving a finger. The device 100 also comprises a lancing device 104 connected to the body. The lancing device 104 comprises a lancet or lancing member within it which is arranged to lance into the space within the body 102. The lancing device 100 also comprises a lancing member actuator for activating the lancing member. The device 100 also comprises a capillary collection device 106. One end of the capillary collection device 106 is arranged to open into the chamber within the body 102.

The device 100 can be used to obtain a blood sample from a finger. To do this, the end of a finger is inserted into the chamber and the lancing member actuator is activated by pressing its end so that the lancing member inside it pierces the skin of the end of the finger within the chamber. The finger is then turned so that the blood touches the end of the capillary collection within the chamber so that the blood is drawn into the capillary collection device 106 by capillary action.

The lancet or lancing member may be provided as a separate, disposable component.

The device need not comprise a capillary collection device. Such a configuration can be used as a finger lancing device (as opposed to a blood collection device). In such a configuration, the finger lancing device may be used to prick the finger before removing the finger. In this way, the blood can be applied to a separate transfer or collection device such as a capillary collection device, e.g. a capillary tube.

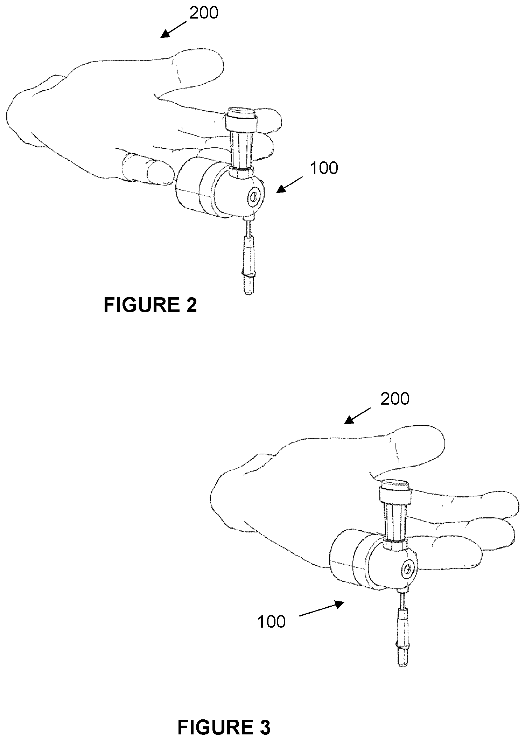

Figure 2 illustrates a hand 200 next to the blood collection device 100 before the little finger of the hand is inserted into the device 100. The body 102 of the device has an opening to the chamber inside the body into which the finger is inserted.

Figure 3 illustrates the hand 200 and device 100 with the little finger of the hand inserted into or received within the chamber.

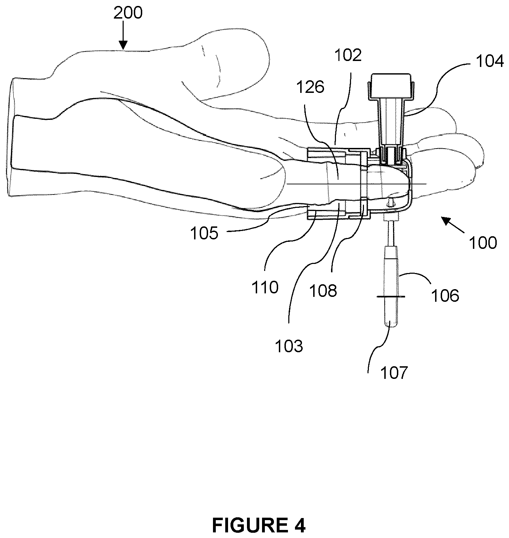

Figure 4 is a cross-sectional side view of the blood collection device 100 and hand 200 in which the little finger 126 has been inserted into the chamber 103 of the body 102. The figure shows the little finger 126 in a position where the lancing device 104 can be activated to prick the finger.

The body 102 is generally circular in cross section and the chamber 103 or space inside the body for receiving the finger is generally cylindrical in shape.

A wipe 110 is provided within the chamber 103 on a wall of the chamber. The wipe is for wiping the finger before lancing. As the finger is inserted into the chamber 103 (before reaching the position depicted) the wipe 110 is used to wipe the finger clean. The wipe 110 is provided within the chamber on a wall of the chamber between the opening 105 and the point where the lancing member will prick the finger so that, as the finger enters the chamber 103, the finger is wiped on wipe 110. The wipe 110 can be positioned in any suitable position for wiping the finger, and in this example is provided near the opening. The wipe 110 in this example is a 70% alcohol wipe.

An elastomeric ring 108 is provided within the chamber on a wall of the chamber. The ring 108 helps guide the finger and can provide an interference fit to accommodate different finger sizes. In a configuration with an elastomeric ring, the body 102 may be made of a rigid material such as plastic, though other less rigid/flexible materials may be used.

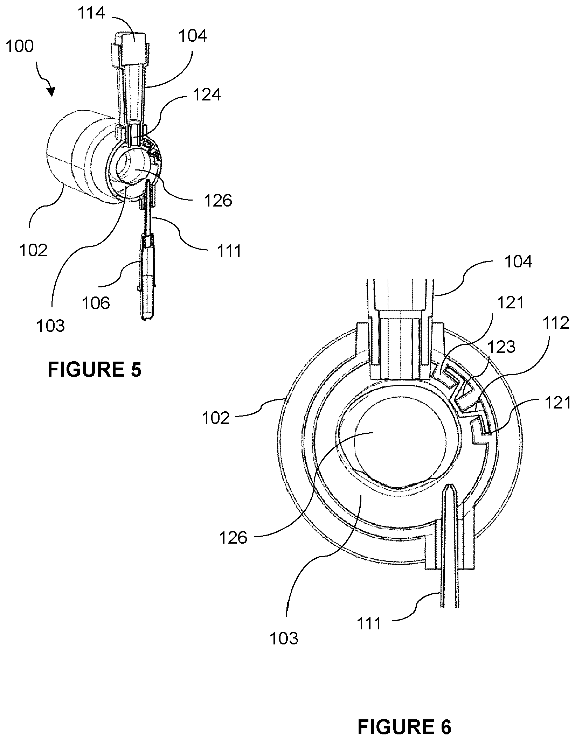

Figure 5 is a cross-sectional perspective view of the device 100. The cross section has been taken along a plane cutting through the body 102, chamber 103, lancing device 104 and capillary collection device 106. Figure 6 is a cross-sectional end view of the device 100 which uses the same cross section. Figure 6 is a magnified view when compared to Figure 5 and does not show the whole lancing device 104 or the whole capillary collection device 106.

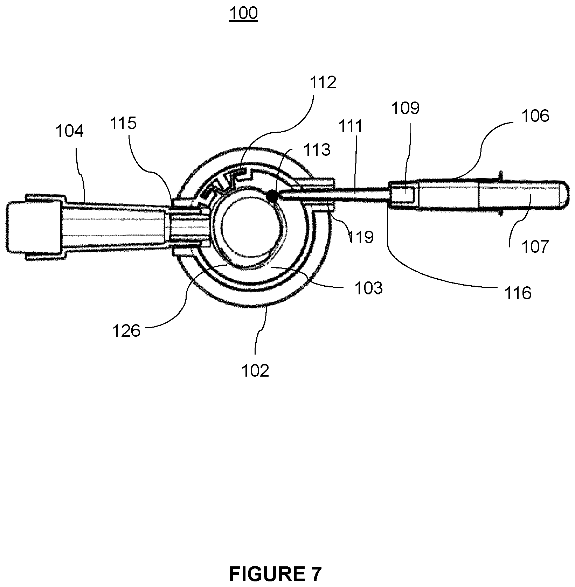

Referring to Figure 5, the device 100 is shown with the body 102, chamber 103, lancing device 104 and capillary collection device 106. As illustrated, the lancing device 104 comprises a lancing member actuator 114 and a lancing member 124 (or lancet). Finger 126, which has been inserted into the chamber 103 can be pricked by pressing the actuator 24 which is arranged to drive the lancing member towards the finger so that the lancing member pricks the finger. The capillary collection device comprises a capillary tip 111 and is described in more detail below with reference to Figure 7.

Lancing devices such as lancing device 104 are known and are commercially available. One example lancing device is the Accu-Chek^{™} Safe-T Pro Plus lancing device available from Roche Diagnostics, a division of F Hoffmann-La Roche AG. Embodiments may use any lancing device, including commercially available devices, such as those available from Roche Diagnostics, or a specially designed device. The lancing device 104 is connected to the body 102. As can be seen, the lancing device 104 connects to body 102 via a hole 115 for receiving the lancing device 104. In the depicted configuration the lancing device is held in the hole 115 through a press or friction fit. In other configurations, the lancing device 104 can be bonded to the body, for example with a suitable adhesive such as a silicone adhesive.

Referring to Figure 6, an absorbent wipe 112 is provided on the wall of the chamber between the lancing device 104 and the capillary collection device 106. More specifically, the wipe is provided inside the chamber on the wall of the chamber between the position where the lancing member enters the chamber and the position where the opening of one end of the capillary tip of the capillary collection device connects to the chamber. As can be seen, the wipe 112 is held in place by L-shaped formations 121 and projection 123. After the finger has been pricked using the lancing device, the finger can be rotated within the chamber in the direction of the wipe 112. The first drop of blood can be absorbed by the wipe 112. The finger can then be rotated further in the same direction so that blood from the lanced finger comes into contact with the end of the end of capillary tip 111 of the capillary collection device 106. Blood is then drawn into the capillary collection device.

Referring to Figure 7, the figure shows the finger in a position where a drop of blood 113 from the lanced site on the finger has been brought into contact with the end of the capillary tip 111 of the capillary collection device 106.

As mentioned above, the capillary collection device 106 comprises a capillary tip 111. One end of the capillary tip fits into a hole 119 in the body 102 and at the other end there is a fill line 116 to indicate a volume of blood required. The device 106 also comprises a filter at the end of the capillary tip, just beyond the fill line. The device also comprises a piston 107 that sits within a piston housing. The piston 107 is provided with a ventilation hole at the end. After the capillary tip 111 of the collection device is filled to the fill line with blood, the device can be removed from the body 102 and the piston 107 can be used to dispense the blood sample.

Capillary collection devices such as the capillary collection device 106 are known and are commercially available. One example capillary collection device is the Minivette^{™} POCT collection device available from Sarstedt AG & Co. Embodiments may use any capillary collection device, including commercially available devices, such as those available from Sarstedt AG & Co, or a specially designed device. The capillary collection device 106 is connected to the body 102. As can be seen, the capillary collection device 106 connects to body 102 via a hole 119 for receiving the lancing device 104. In the depicted configuration the capillary collection device is held in the hole 119 through a press or friction fit. In other configurations, other ways of connecting the components may be used.

In some configurations the capillary and fill area are visible so the operator can see the blood flow in and know whether any 'milking' of the finger to express further blood is needed.

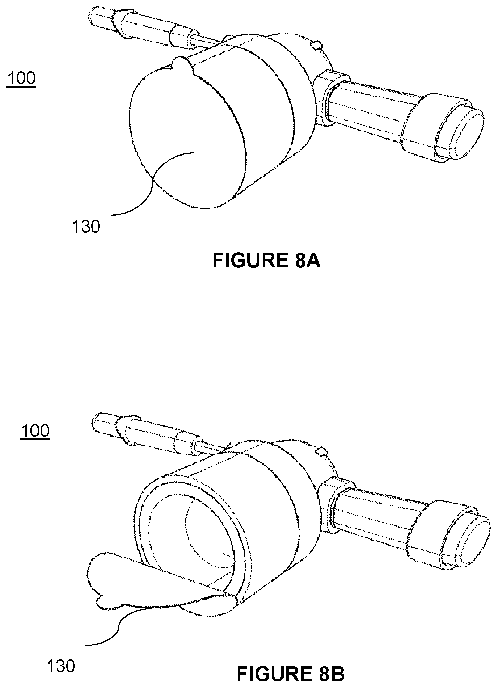

The device 100 can be provided with an openable seal which seals the opening of the chamber 103. Figures 8A and B depict perspective views of the device 100 with an openable seal 130. In Figure 8A the seal is sealed and in Figure 8B the seal is depicted in a near removed position. In this embodiment the seal 130 is a peelable film seal that is applied to the body chamber to keep the device sealed from the external environment and to help prevent the wipe 110 drying out. The seal can be made of any commercially available sealing film such as a medical grade sealing film (e.g. a plastic film, typically a polyester film). An adhesive such as a medical grade silicone or acrylic adhesive can be used to adhere the seal 130 to the body 102. The wipe 110 can be provided on the inside of the openable seal instead of being provided on a wall of the chamber. Wipes in both locations can be provided in some configurations.

According to the invention, the body 102 is flexible. That is, the body is made from a flexible material so that further drops of blood can be expressed from the finger 126 through the body 102. Such configurations may not include an elastomeric ring.

In some configurations, the body 102 is transparent.

The body 102 may be made of any suitable material. In some configurations, the body is made from a silicone, urethane or any medical grade elastomer. The body can be made in any suitable way. One way of making the body is using a mould.

The body 102 has been described in connection with the lancing device 104 and the capillary collection device 106. The body can be made as a separate component and later connected to the lancing device and the capillary collection device. The body 102 can be produced and sold separately, for the user to assemble with a lancing device and/or a capillary collection device that they have sourced separately. The finger lancing device 100 may be pre-assembled and sold in pre-assembled form. The finger lancing device 100 may be sold as a kit of parts comprising the body and one or both of the lancing device 104 and capillary collection device 116. The holes 115 and 119 may each be sealed with a removable seal when the body is provided on its own or as a kit of unassembled parts. The seals can be made of any commercially available sealing film such as a medical grade sealing film (e.g. a plastic film, typically a polyester film). An adhesive such as a medical grade silicone or acrylic adhesive can be used to adhere the seals to the body 102.

The lancing device and capillary collection device can be commercially available devices such as those referenced above. The lancing device and capillary collection device are typically disposable components. The body of the finger lancing device/blood collection device may also be disposable or may be used more than once, particularly in a simple embodiment without any one-use components.

The embodiments of the invention shown in the drawings and described above are exemplary embodiments only and are not intended to limit the scope of the appended claims. Various modifications are possible and will be readily apparent to the skilled person in the art. It is intended that any combination of non-mutually exclusive features described herein are within the scope of the present invention. That is, features of the described embodiments can be combined with any appropriate aspect described above and optional features of any one aspect can be combined with any other appropriate aspect.

## Claims

1. A finger lancing device (100) comprising:
a body (102) having a chamber (103) for receiving a finger; and
a lancing device (104) comprising a lancing member and a lancing member actuator configured to actuate the lancing member to lance a finger received in the chamber, wherein the body is flexible and made from an elastomeric material, and wherein the lancing member is arranged to lance into the chamber.

2. A finger lancing device according to claim 1, further comprising a wipe (110) provided within the chamber for wiping the finger before lancing, and optionally wherein the wipe is provided on a wall of the chamber, optionally near an end of the chamber for receiving the finger.

3. A finger lancing device according to any preceding claim, further comprising an absorbent wipe (112) provided on a wall of the chamber for wiping away a first drop of blood.

4. A finger lancing device according to any preceding claim, further comprising an openable seal (130), the openable seal being at an end of the chamber for receiving the finger.

5. A finger lancing device according to claim 4 when dependent on claim 2, wherein the wipe is provided on the openable seal, optionally on the inner side of the openable seal.

6. A finger lancing device according to any preceding claim, further comprising a capillary collection device (106) for collecting a blood sample from the finger received in the chamber, and optionally wherein the capillary collection device has a fill line (116) to indicate a volume of blood required.

7. A finger lancing device according to any preceding claim, wherein the body is transparent.

8. A body (102) for a finger lancing device comprising a chamber for receiving a finger, wherein the body comprises a hole (115) for receiving a lancing device,
wherein the body is flexible and made from an elastomeric material, and
wherein an absorbent wipe (112) is provided on a wall of the chamber for wiping away a first drop of blood.

9. A body according to claim 8, further comprising an openable seal covering the hole for receiving the lancing device.

10. A body according to claim 8 or claim 9, wherein the body further comprises a hole (119) for receiving a capillary collection device.

11. A body according to claim 10, further comprising an openable seal covering the hole for receiving the capillary collection device.

12. A body according to any of claims 8 to 11, further comprising an openable seal (130), the openable seal being at an end of the chamber for receiving the finger.

13. A body according to claim 12, wherein a wipe (110) for wiping the finger before lancing is provided on the openable seal, optionally on the inner side of the openable seal.

14. A kit comprising a body according to any of claims 8 to 13 and at least one of a lancing device or a capillary collection device.

## Patentansprüche

1. Fingerstechhilfe (100), umfassend:
einen Körper (102) mit einer Kammer (103) zur Aufnahme eines Fingers und
eine Stechhilfe (104), die ein Stechglied und einen Stechgliedaktuator umfasst, der zur Betätigung des Stechglieds ausgestaltet ist, um in einen in der Kammer aufgenommenen Finger zu stechen,
wobei der Körper flexibel und aus einem elastomeren Material hergestellt ist und wobei das Stechglied zum Einstechen in die Kammer angeordnet ist.

2. Fingerstechhilfe nach Anspruch 1, ferner umfassend eine in der Kammer vorgesehene Abwischvorrichtung (110) zum Abwischen des Fingers vor dem Einstechen, und optional wobei die Abwischvorrichtung an einer Wand der Kammer vorgesehen ist, optional in der Nähe eines Endes der Kammer zur Aufnahme des Fingers.

3. Fingerstechhilfe nach einem der vorhergehenden Ansprüche, ferner umfassend eine absorbierende Wischvorrichtung (112), die an einer Wand der Kammer vorgesehen ist, um einen ersten Tropfen Blut wegzuwischen.

4. Fingerstechhilfe nach einem der vorhergehenden Ansprüche, ferner umfassend eine zu öffnende Abdichtung (130), wobei die zu öffnende Abdichtung an einem Ende der Kammer zur Aufnahme des Fingers angeordnet ist.

5. Fingerstechhilfe nach Anspruch 4, wenn von Anspruch 2 abhängig, wobei die Wischvorrichtung an der zu öffnenden Abdichtung vorgesehen ist, optional an der Innenseite der zu öffnenden Abdichtung.

6. Fingerstechhilfe nach einem der vorhergehenden Ansprüche, ferner umfassend eine Kapillarsammelvorrichtung (106) zum Sammeln einer Blutprobe von dem in der Kammer aufgenommenen Finger, und optional wobei die Kapillarsammelvorrichtung eine Füllmarkierungslinie (116) zur Angabe eines erforderlichen Blutvolumens hat.

7. Fingerstechhilfe nach einem der vorhergehenden Ansprüche, wobei der Körper transparent ist.

8. Körper (102) für eine Fingerstechhilfe, umfassend eine Kammer zur Aufnahme eines Fingers,
wobei der Körper ein Loch (115) zur Aufnahme einer Stechhilfe umfasst, wobei der Körper flexibel und aus einem elastomeren Material hergestellt ist und
wobei an einer Wand der Kammer eine absorbierende Abwischvorrichtung (112) vorgesehen ist, um einen ersten Tropfen Blut wegzuwischen.

9. Körper nach Anspruch 8, ferner umfassend eine zu öffnende Abdichtung, die das Loch zur Aufnahme der Stechhilfe abdeckt.

10. Körper nach Anspruch 8 oder Anspruch 9, wobei der Körper ferner ein Loch (119) zur Aufnahme einer Kapillarsammelvorrichtung umfasst.

11. Körper nach Anspruch 10, ferner umfassend eine zu öffnende Abdichtung, die das Loch zur Aufnahme der Kapillarsammelvorrichtung abdeckt.

12. Körper nach einem der Ansprüche 8 bis 11, ferner umfassend eine zu öffnende Abdichtung (130), wobei die zu öffnende Abdichtung an einem Ende der Kammer zur Aufnahme des Fingers angeordnet ist.

13. Körper nach Anspruch 12, wobei eine Abwischvorrichtung (110) zum Abwischen des Fingers vor dem Einstechen an der zu öffnenden Abdichtung, optional an der Innenseite der zu öffnenden Abdichtung, vorgesehen ist.

14. Satz, umfassend einen Körper nach einem der Ansprüche 8 bis 13 und mindestens eine Fingerstechhilfe oder eine Kapillarsammelvorrichtung.

## Revendications

1. Dispositif de piquage du doigt (100) comprenant :
un corps (102) comportant une chambre (103) destinée à recevoir un doigt ; et
un dispositif de piquage (104) comprenant un élément de piquage et un actionneur d'élément de piquage conçu pour actionner l'élément de piquage pour piquer un doigt reçu dans la chambre,
dans lequel le corps est flexible et constitué d'un matériau élastomère, et dans lequel l'élément de piquage est disposé pour piquer dans la chambre.

2. Dispositif de piquage du doigt selon la revendication 1, comprenant, en outre, une lingette (110) placée à l'intérieur de la chambre pour essuyer le doigt avant le piquage, et dans lequel, éventuellement, la lingette est placée sur une paroi de la chambre, éventuellement à proximité d'une extrémité de la chambre destinée à recevoir le doigt.

3. Dispositif de piquage du doigt selon l'une quelconque des revendications précédentes, comprenant, en outre, une lingette absorbante (112) placée sur une paroi de la chambre pour essuyer une première goutte de sang.

4. Dispositif de piquage du doigt selon l'une quelconque des revendications précédentes, comprenant, en outre, un élément de scellage ouvrable (130), l'élément de scellage ouvrable étant situé à une extrémité de la chambre destinée à recevoir le doigt.

5. Dispositif de piquage du doigt selon la revendication 4 lorsqu'elle dépend de la revendication 2, dans lequel la lingette est placée sur l'élément de scellage ouvrable, éventuellement sur le côté intérieur de l'élément de scellage ouvrable.

6. Dispositif de piquage du doigt selon l'une quelconque des revendications précédentes, comprenant, en outre, un dispositif de prélèvement capillaire (106) servant à prélever un échantillon de sang dans le doigt reçu dans la chambre, et dans lequel, éventuellement, le dispositif de prélèvement capillaire comporte une ligne de remplissage (116) pour indiquer un volume de sang requis.

7. Dispositif de piquage du doigt selon l'une quelconque des revendications précédentes, dans lequel le corps est transparent.

8. Corps (102) pour un dispositif de piquage du doigt comprenant une chambre destinée à recevoir un doigt,
le corps comprenant un trou (115) destiné à recevoir un dispositif de piquage, le corps étant flexible et constitué d'un matériau élastomère, et
une lingette absorbante (112) étant placée sur une paroi de la chambre pour essuyer une première goutte de sang.

9. Corps selon la revendication 8, comprenant, en outre, un élément de scellage ouvrable recouvrant le trou destiné à recevoir le dispositif de piquage.

10. Corps selon la revendication 8 ou la revendication 9, le corps comprenant, en outre, un trou (119) destiné à recevoir un dispositif de prélèvement capillaire.

11. Corps selon la revendication 10, comprenant, en outre, un élément de scellage ouvrable recouvrant le trou destiné à recevoir le dispositif de prélèvement capillaire.

12. Corps selon l'une quelconque des revendications 8 à 11, comprenant, en outre, un élément de scellage ouvrable (130), l'élément de scellage ouvrable étant situé à une extrémité de la chambre destinée à recevoir le doigt.

13. Corps selon la revendication 12, dans lequel une lingette (110) servant à essuyer le doigt avant le piquage est placée sur l'élément de scellage ouvrable, éventuellement sur le côté intérieur de l'élément de scellage ouvrable.

14. Matériel comprenant un corps selon l'une quelconque des revendications 8 à 13 et un dispositif de piquage et/ou un dispositif de prélèvement capillaire.
